# EUROPEAN PATENT SPECIFICATION

(11) **EP 3 271 020 B1**
(45) Date of publication and mention of the grant of the patent: **25.11.2020**
(21) Application number: 16765468.0
(22) Date of filing: 10.03.2016
(51) Int. Cl.: A61P 31/00, A61P 31/12, A61P 31/14, A61K 31/551, A61K 38/17

(54) **REVERSAL OF LATENCY OF RETROVIRUSES WITH A GALECTIN PROTEIN**
UMKEHRUNG DER LATENZ VON RETROVIREN MIT GALECTIN-PROTEIN
INVERSION DE LA LATENCE DE RÉTROVIRUS AVEC UNE PROTÉINE GALECTINE

(30) Priority: 18.03.2015 US 201562135075 P
(43) Date of publication of application: 24.01.2018
(73) Proprietor: Vitalant, San Francisco, CA 94118 (US); University of Hawaii, Honolulu, Hawaii 96822-2216 (US); GalPharma Co., Ltd., Kagawa, 761-0301 (JP)
(72) Inventor: PILLAI, Satish, K., San Francisco, CA 94118 (US); ABDEL-MOHSEN, Mohamed, San Francisco, CA 94118 (US); CHAVEZ, Leonard, San Francisco, CA 94118 (US); NDHLOVU, Lishomwa, Honolulu, HI 96822-2385 (US); TANDON, Ravi, Honolulu, HI 96822-2385 (US); NIKI, Toshiro, Takamatsu-shi Kagawa 761-0301 (JP); HIRASHIMA, Misuomi, Takamatsu-shi Kagawa 761-0301 (JP)
(74) Representative: Cornish, Kristina Victoria Joy
(86) International application number: PCT/US2016/021831
(87) International publication number: WO 2016/149045

(56) References cited:
- EP-A1- 1 736 541
- EP-A1- 2 650 361
- WO-A2-2010/099169
- WO-A2-2011/100528
- WO-A2-2012/051492
- WO-A2-2014/201426
- US-A1- 2011 305 662
- US-A1- 2012 328 568
- MOHAMED ABDEL-MOHSEN ET AL: "Human Galectin-9 Is a Potent Mediator of HIV Transcription and Reactivation", PLOS PATHOGENS, vol. 12, no. 6, 2 June 2016 (2016-06-02), page e1005677, XP55506174, DOI: 10.1371/journal.ppat.1005677
- BULLEN ET AL.: 'New ex vivo approaches distinguish effective and ineffective single agents for reversing HIV-1 latency in vivo.' NATURE MEDICINE vol. 20, no. 4, April 2014, pages 425 - 429, XP055312788
- MERCIER ET AL.: 'Galectin-1 promotes HIV-1 infectivity in macrophages through stabilization of viral adsorption.' VIROLOGY vol. 371, no. 1, 05 February 2008, pages 121 - 129, XP022417957
- ELAHI ET AL.: 'Galectin-9 binding to Tim-3 renders activated human CD 4+ T cells less susceptible to HIV-1 infection.' BLOOD vol. 119, no. 18, 03 May 2012, pages 4192 - 4204, XP055312789
- REDDY ET AL.: 'Influence of galectin-9/Tim-3 interaction on herpes simplex virus-1 latency' J IMMUNOL vol. 187, no. 11, 01 December 2011, pages 5745 - 5755, XP055312790
- LAIRD ET AL.: 'Ex vivo analysis identifies effective HIV-1 latency-reversing drug combinations.' J CLIN INVEST vol. 125, no. 5, 30 March 2015, pages 1901 - 1912, XP055312791

## Description

### FIELD OF THE INVENTION

The present disclosure relates to generally to the treatment of subjects infected with retroviruses and specifically to the treatment of HIV infected subjects using a recombinant galectin protein. The present invention relates generally to the treatment of subjects infected with HIV using a galectin-9 protein

### BACKGROUND OF THE INVENTION

HIV-1, the causative agent of AIDS, infects 35 million people worldwide and nearly 1.2 million persons in the United States. The advent of antiretroviral therapy (ART) has dramatically reduced morbidity and mortality for HIV-infected individuals with access to healthcare in resource-rich countries. However, despite years of potent therapy, eradication of infection is not achieved with conventional treatment strategies, necessitating lifelong ART. Moreover, accumulating data suggest that "non-AIDS" cardiovascular, renal and hepatic diseases are amplified by HIV infection, and the immune system may exhibit premature senescence even among patients with viral suppression. Although enormous progress has been made to provide ART in resource limited settings, there are huge economical, political and operational challenges to reach the goal of universal access to lifelong treatment. These realities have created a pronounced interest in developing strategies to eradicate HIV-1 in infected individuals.

It has been shown that despite long periods of suppressive antiretroviral therapy, a population of latently infected cells persists which is capable of producing replication competent virus upon *ex vivo* activation. These cells are comprised chiefly of resting memory CD4+ T lymphocytes and cells of the monocyte-macrophage lineage are believed to harbor latent virus as well. Rebounding plasma HIV RNA levels following discontinuation of antiretroviral therapy even after prolonged suppression of plasma viremia confirm the *in vivo* significance of this reservoir. In one of the largest studies to examine the half-life of the circulating CD4+ reservoir involving 62 HIV-infected adults with up to 7 years of viral suppression on HAART, frequencies of latently-infected T cells remained between 0.03-3 infectious units per million resting CD4+ T cells, with no significant decline over time. The total reservoir size was estimated to be 10⁶ cells with a half-life of 44.2 months, yielding an estimated reservoir eradication time of 73.2 years, suggesting that clearance is not achieved spontaneously on a relevant time scale.

Elimination of the HIV-1 latent reservoir is critical to achieving HIV-1 eradication *in vivo.* The "shock and kill" strategy is currently the most widely discussed approach to eliminating the viral reservoir. In this approach, drugs are administered to reverse HIV-1 latency and induce viral production, ultimately resulting in the death of infected cells by direct viral cytopathic effects or immune-mediated clearance. Latency reversing agents are administered during suppressive antiretroviral therapy (ART), thereby preventing reactivated virus from replenishing the reservoir through infection of new cells. Although the theory behind this approach is intuitive and elegant, to date, the shock and kill strategy has only achieved limited success. Clinical trials involving latency reversing agents (LRAs) such as vorinostat and disulfiram have failed to demonstrate significant reduction in reservoir size, although transient elevation in viral RNA has been observed. Accordingly, *in vitro* experiments have revealed that the majority of existing LRAs exert weak effects on HIV-1 transcription and reactivation. The future success of shock and kill will depend on our capacity to design highly efficacious new LRAs and/or adjuvant therapies to boost the reactivation potential of existing LRAs.
Mercier et al., 2008, Virology Volume 371, Issue 1, Pages 121-129 discloses that Galectin-1 promotes HIV-1 infectivity in macrophages through stabilization of viral adsorption. Laird et al., 2015, J Clin Invest. 125(5):1901-191 discloses HIV-1 latency-reversing drug combinations.

### SUMMARY OF THE INVENTION

The present invention provides a galectin-9 protein for use in a method of treating HIV infection by reversing human immunodeficiency virus (HIV) latency in a cell, the method comprising administering the galectin protein to a subject.

The present invention also provides a pharmaceutical composition comprising a galectin-9 protein and a pharmaceutically acceptable carrier for use in a method of treating HIV infection by reversing human immunodeficiency virus (HIV) latency in a cell.

The present invention relates to the seminal discovery of methods of using recombinant galectin proteins to reverse HIV latency. Specifically, the present invention relates to the use of recombinant galectin-9 (rGal-9) to reactivate latent HIV, wherein the HIV is hypermutated rendering the virus replication incompetent. Further, rGal-9 induces the expression of APOBEC3 cytidine deaminases and NFκB pathway components and decreases histone deacetylase (HDAC) expression.

In one embodiment, the present invention provides a method of reversing human immunodeficiency virus (HIV) latency in a cell comprising administering a galectin protein to a subject in need thereof, thereby reversing the HIV latency. In one aspect of the disclosure, the galectin protein is galectin-1, galectin-2, galectin-3, galectin-4, galectin-5, galectin-6, galectin-7, galectin-8, galectin-10, galectin-11, galectin-12, galectin-13, galectin-14 or galectin-15. In a specific aspect of the invention, the galectin protein is galectin-9 (rGal-9). In another aspect, after administration of the galectin protein the HIV is hypermutated. In a further aspect, the hypermutated HIV is replication incompetent. In an additional aspect, expression of at least one APOBEC3 cytidine deaminase and at least one NFκB pathway component is induced. In one aspect, the induction of expression of the at least one APOBEC3 cytidine deaminase and the at least one NFκB pathway component is at least two fold above normal physiologic levels. In specific aspects, the at least one APOBEC3 cytidine deaminase is APOBEC3B, APOBEC3F, APOBEC3G, APOBEC3H or a combination thereof and the at least one NFκB pathway component is NFκB1, NFκB2, NFκBIA, NFκBID, NFκBIE, NFκB1, NFκBIL1, NFκBIZ, NFRκB, REL, RELA, RELB or a combination thereof. In another aspect, the expression of at least one histone deacetylase (HDAC) protein is decreased compared to normal physiologic levels. In specific aspects, the at least one HDAC protein is HDAC1, HDAC2, HDAC3 or a combination thereof.

In a further embodiment, the present disclosure provides a method of treating a subject with a retrovirus infection comprising administering recombinant galectin-9 (rGal-9) to the subject, thereby treating the retroviral infection. In one aspect, the retrovirus is HIV-1, HIV-2, HTLV-1, HTLV-2, HTLV-3, HTLV-4 or a combination thereof. In a specific aspect, the retrovirus is HIV-1 or HIV-2. In another aspect, after administration of rGal-9 the HIV is hypermutated. In a further aspect, the hypermutated HIV is replication incompetent. In an additional aspect, expression of at least one APOBEC3 cytidine deaminase and at least one NFκB pathway component is induced. In one aspect, the induction of expression of the at least one APOBEC3 cytidine deaminase and the at least one NFκB pathway component is at least two fold above normal physiologic levels. In specific aspects, the at least one APOBEC3 cytidine deaminase is APOBEC3B, APOBEC3F, APOBEC3G, APOBEC3H or a combination thereof and the at least one NFκB pathway component is NFκB1, NFκB2, NFκBIA, NFκBID, NFκBIE, NFκB1, NFκBIL1, NFκBIZ, NFRκB, REL, RELA, RELB or a combination thereof. In another aspect, the expression of at least one histone deacetylase (HDAC) protein is decreased compared to normal physiologic levels. In specific aspects, the at least one HDAC protein is HDAC1, HDAC2, HDAC3 or a combination thereof. In a further aspect, an anti-retroviral therapeutic is administered. In one aspect, the anti-retroviral therapeutic is enfuvirtide, zidovudine, abacavir, lamivudine, emtricitabine, tenfovir, nevirpine, efavirenz, etravirine, rilpivirine, raltegravir, elvitegravir, dolutegravir, lopinavir, indinavir, nelfinavir, amprenavir, ritonavir, darunavir, atazanavir, bevirimat, vivecon, stavudine, didanosine, delavirdine, nevirapine, fosamprenavir, saquinavir, tipranavir, maraviroc or a combination thereof. In another aspect, the anti-retroviral therapeutic is a nucleoside/nucleotide reverse transcriptase inhibitor (NRTI), non-nucleoside reverse transcriptase inhibitor (NNRTI), protease inhibitor, fusion or entry inhibitor, integrase inhibitor or a combination thereof.

In an additional embodiment, the present invention provides pharmaceutical compositions comprising a recombinant galectin protein and a pharmaceutically acceptable carrier. In one aspect of the disclosure, the recombinant galectin protein is galectin-1, galectin-2, galectin-3, galectin-4, galectin-5, galectin-6, galectin-7, galectin-8, galectin-10, galectin-11, galectin-12, galectin-13, galectin-14 or galectin-15. In a specific aspect of the invention, the recombinant galectin protein is galectin-9 (rGal-9). Illustrative rGal-9 is described in U.S. Patent No. 8,268,324.

### BRIEF DESCRIPTION OF THE DRAWINGS

**Figure** 1 shows Spearman's correlation between CD4+ T cell-associated HIV RNA and expression of the p21 host restriction factor in HIV-infected, ART-suppressed individuals.
**Figures 2A-2B** show the *in vitro* reactivation of HIV expression by recombinant galectin-9 in the J-Lat latency model. **(A)** Dose response of HIV reactivation by rGal-9 after 2, 6, 12, and 24 hours of stimulation. **(B)** Effects of combining 100nM of rGal-9 and aCD3/aCD28 stimulation after 24 hours of stimulation.
**Figure 3** depicts a schematic of workflow to evaluate effects of rGal-9 treatment on the human transcriptome.
**Figure 4** shows a heat map describing effects of rGal-9 on anti-HIV cell-intrinsic immunity.
**Figure 5** shows the induction of APOBEC3G host cytidine deaminase in HIV latently-infected cells by rGal-9.
**Figure 6** shows the induction of multiple NFkB pathway components by rGal-9.
**Figure 7** shows rGal-9 selectively inhibits histone deacetylases (HDACs).
**Figures 8A-8B** show that freshly-isolated primary CD4+ T cells tolerate galectin-9 treatment at least up to 200nM concentration. **(A)** Percentage of live CD4+ T cells from three healthy donors treated with either 0.5% DMSO or escalating concentrations of galectin-9. **(B)** A representative set of the flow cytometry data.

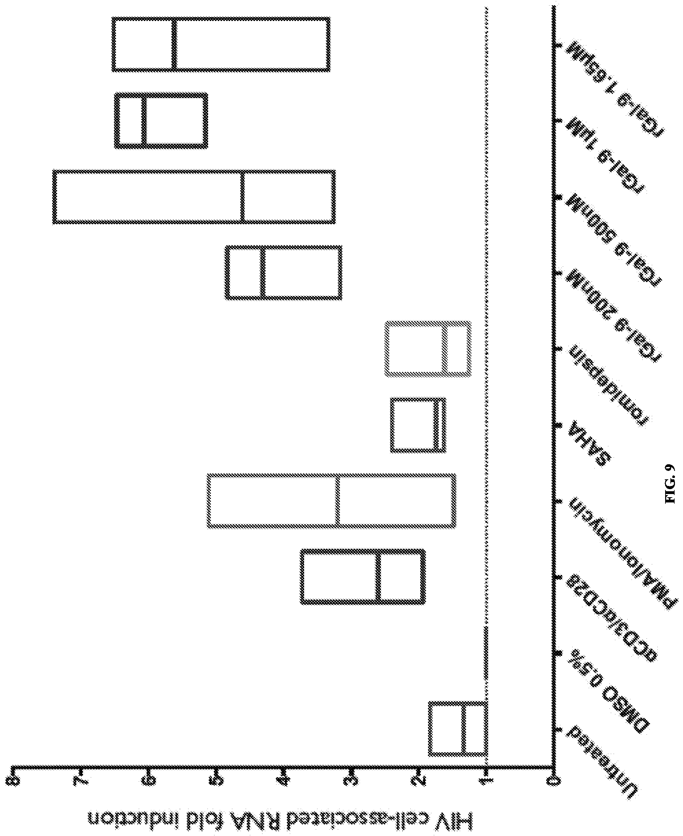

**Figure 9** shows the potent *ex vivo* reactivation of latent HIV by rGal-9.

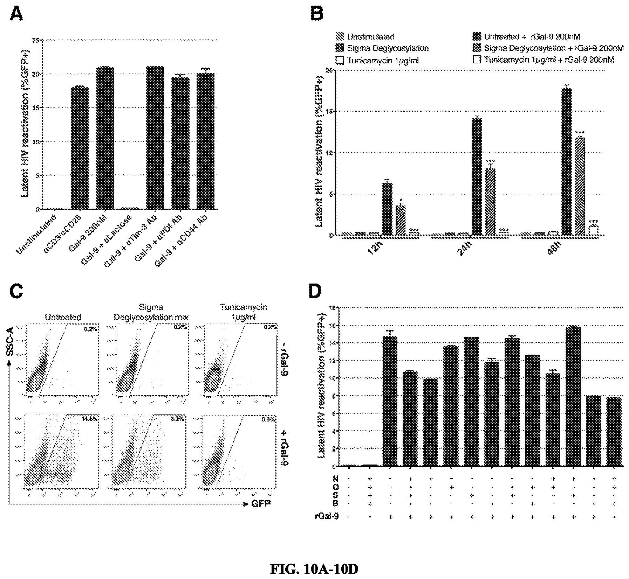

**Figures 10A-10D** show graphs and a flow cytometry analysis to show rGal-9 induces HIV transcription and reactivation in a glycan-dependent manner. Figure 10A-Effects of anti-Tim-3 antibody, anti-CD44 antibody, or anti-PDI antibody administration on rGal-9-mediated reactivation of HIV in J-Lat 5A8 cells. Antibodies were added 30 minutes prior to administration of 200nM rGal-9. α-lactose was used as a positive control. Figures 10B, C- Treatment of J-Lat 5A8 cells with either 1 µg/ml tunicamycin, or with an enzymatic deglycosylation mix for 24 h prior to rGal-9 stimulation. J-Lat cells were analyzed by flow cytometry to assess HIV-encoded GFP expression. Statistical comparisons were performed using two-tailed Mann-Whitney tests. Figure 10D- Effects of deglycosylation enzyme combinations on rGal-9-mediated HIV latency reversal in J-Lat 5A8 cells. N = PNGase F (Elizabethkingia miricola); O = O-Glycosidase (recombinant from Streptococcus pneumonia; S = α-(2→3,6,8,9)-Neuraminidase (recombinant from Arthrobacter ureafaciens); B = β (1→4)-Galactosidase (recombinant from Streptococcus pneumonia) + β-N-Acetylglucosaminidase (recombinant from Streptococcus pneumonia). Mean ± SEM is displayed.

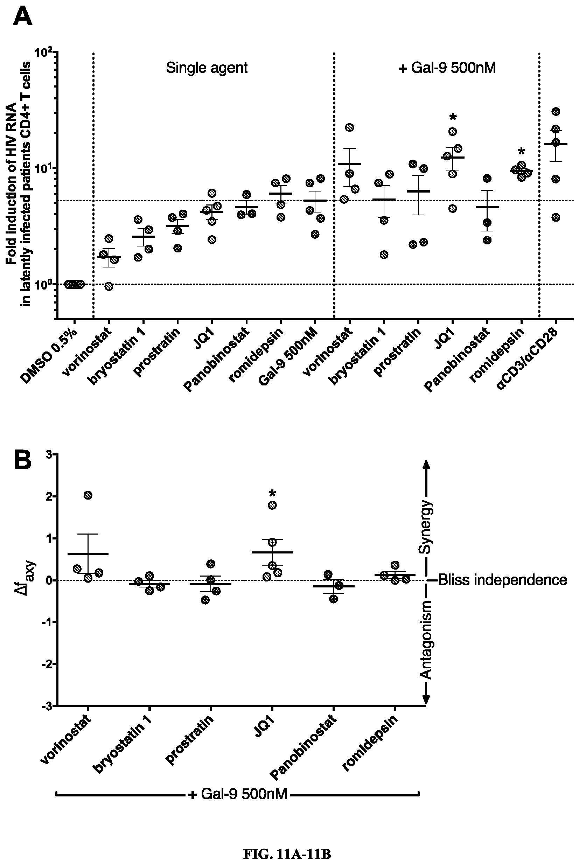

**Figures 11A and 11B** provide data showing that Galectin-9 synergizes with JQ1 in reactivating latent HIV. Figure 11A- CD4+ T cells from HIV-infected ART-suppressed individuals were treated with 500nM of rGal-9, 1 µM vorinostat, 10nM bryostatin, 300nM prostratin, 1µM JQ1, or 30nM panobinostat alone or in combination with 500nM of rGal-9 for 24 hours, and fold induction of cell-associated HIV RNA was determined using quantitative real-time PCR. * = p<0.05 compared with Gal-9 500nM treatment alone. Figure 11 B- The Bliss independence model was utilized for calculation of synergy for drug combinations. Δfaxy = 0 signifies pure additive effect. Δfaxy>0 signifies Synergy, while Δ faxy<0 signifies antagonism. Statistical significance was calculated using a one tailed paired t-test comparing predicted and observed drug combination effects. * = p < 0.05.

### DETAILED DESCRIPTION OF THE INVENTION

Before the present compositions and methods are described, it is to be understood that this invention is not limited to particular compositions, methods, and experimental conditions described, as such compositions, methods, and conditions may vary. It is also to be understood that the terminology used herein is for purposes of describing particular embodiments only. The invention is defined in the claims.

Unless defined otherwise, all technical and scientific terms used herein have the same meaning as commonly understood by one of ordinary skill in the art to which this invention belongs. Although any methods and materials similar or equivalent to those described herein can be used in the practice or testing of the invention, the preferred methods and materials are now described. The definitions set forth below are for understanding of the disclosure but shall in no way be considered to supplant the understanding of the terms held by those of ordinary skill in the art.

As used in this specification and the appended claims, the singular forms "a", "an", and "the" include plural references unless the context clearly dictates otherwise. Thus, for example, references to "the method" includes one or more methods, and/or steps of the type described herein which will become apparent to those persons skilled in the art upon reading this disclosure and so forth.

As used herein, the terms "treating" or "treatment" or "alleviation" refers to therapeutic treatment, prophylactic and/or preventative measures, wherein the object is to prevent or slow down (lessen) the targeted pathologic condition or disorder. Those in need of treatment include those already with the disorder as well as those prone to have the disorder or those in whom the disorder is to be prevented.

As used herein, the term "treatment" of any disease mentioned herein encompasses an alleviation of at least one symptom of the disease, a reduction in the severity of the disease, or the delay or prevention of disease progression to more serious symptoms that may, in some cases, accompany the disease or lead to at least one other disease (e.g., reduction of viral load). Treatment need not mean that the disease is totally cured. A useful therapeutic agent needs only to reduce the severity of a disease, reduce the severity of one or more symptoms associated with the disease or its treatment, or delay the onset of more serious symptoms or a more serious disease that can occur with some frequency following the treated condition.

As used herein, the terms "effective amount" or "therapeutically effective amount" of a drug used to treat a disease is an amount that can reduce the severity of a disease, reduce the severity of one or more symptoms associated with the disease or its treatment, or delay the onset of more serious symptoms or a more serious disease that can occur with some frequency following the treated condition. An "effective amount" may be determined empirically and in a routine manner, in relation to the stated purpose.

"Polypeptide" and "protein" are used interchangeably herein and refer to a compound of two or more subunit amino acids, amino acid analogs, or other peptidomimetics, regardless of post-translational modification, e.g., phosphorylation or glycosylation. The subunits may be linked by peptide bonds or other bonds such as, for example, ester or ether bonds. Full-length polypeptides, truncated polypeptides, point mutants, insertion mutants, splice variants, chimeric proteins, and fragments thereof are encompassed by this definition. In various embodiments the polypeptides can have at least 10 amino acids or at least 25, or at least 50 or at least 75 or at least 100 or at least 125 or at least 150 or at least 175 or at least 200 amino acids.

The terms "recombinant" or "engineered" as used herein in reference to a nucleic acid molecule, refer to a nucleic acid molecule that has been altered through human intervention. As non-limiting examples, a cDNA is a recombinant DNA molecule, as is any nucleic acid molecule that has been generated by in vitro polymerase reaction(s), or to which linkers have been attached, or that has been integrated into a vector, such as a cloning vector or expression vector. As non-limiting examples, a recombinant nucleic acid molecule: 1) has been synthesized or modified in vitro, for example, using chemical or enzymatic techniques (for example, by use of chemical nucleic acid synthesis, or by use of enzymes for the replication, polymerization, exonucleolytic digestion, endonucleolytic digestion, ligation, reverse transcription, transcription, base modification (including, e.g., methylation), or recombination (including homologous and site-specific recombination)) of nucleic acid molecules; 2) includes conjoined nucleotide sequences that are not conjoined in nature; 3) has been engineered using molecular cloning techniques such that it lacks one or more nucleotides with respect to the naturally occurring nucleic acid molecule sequence; and/or 4) has been manipulated using molecular cloning techniques such that it has one or more sequence changes or rearrangements with respect to the naturally occurring nucleic acid sequence. A "recombinant protein" is a protein produced by genetic engineering, for example, by expression of a genetically engineered nucleic acid molecule in a cell.

As used herein, the term "carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN®, polyethylene glycol (PEG), and PLURONICS®. Further, optional pharmaceutically acceptable carriers, excipients or stabilizers (Remington's Pharmaceutical Sciences 16th edition, Osol, A. Ed. (1980)), in the form of lyophilized formulations or aqueous solutions.

Retroviruses are a family of enveloped viruses that replicate in a host cell through the process of reverse transcription. A retrovirus is a single-stranded positive sense RNA virus with a DNA intermediate and, as an obligate parasite, targets a host cell. Once inside the host cell cytoplasm, the virus uses its own reverse transcriptase enzyme to produce DNA from its RNA genome, the reverse of the usual pattern. This new DNA is then incorporated into the host cell genome by an integrase enzyme, at which point the retroviral DNA is referred to as a provirus. The host cell then treats the viral DNA as part of its own genome, translating and transcribing the viral genes along with the cell's own genes, producing the proteins required to assemble new copies of the virus. It is difficult to detect the virus until it has infected the host. At that point, the infection will persist indefinitely.

Examples of human retroviruses identified include HTLV 1 (T-cell leukaemias/lymphomas, Tropical spastic paraparesis), HTLV 2 (no known pathology), HIV 1 & 2 - AIDS and HTLV-3 and HTLV-4 (no known pathology). All of the human retorviruses infect T cells. HTLV-1 is known to cause a type of cancer called adult T-cell leukemia/lymphoma and a demyelinating disease called HTLV-I associated myelopathy/tropical spastic paraparesis (HAM/TSP). HTLV-2 is a virus closely related to HTLV-I and shares approximately 70% genomic homology with HTLV-I. HTLV-3 and HTLV-4 are two new human retroviruses recently identified which are related to HTLV1 and 2.

The human immunodeficiency virus (HIV) causes the acquired immunodeficiency syndrome (AIDS), a condition in humans in which progressive failure of the immune system allows life-threatening opportunistic infections and cancers to thrive. Without treatment, average survival time after infection with HIV is estimated to be 9 to 11 years, depending on the HIV subtype.

HIV infects vital cells in the human immune system such as helper T cells (specifically CD4+ T cells), macrophages, and dendritic cells. HIV infection leads to low levels of CD4+ T cells through a number of mechanisms, including apoptosis of uninfected bystander cells, direct viral killing of infected cells, and killing of infected CD4+ T cells by CD8 cytotoxic lymphocytes that recognize infected cells. When CD4+ T cell numbers decline below a critical level, cell-mediated immunity is lost, and the body becomes progressively more susceptible to opportunistic infections.

HIV establishes a persistent infection in its host and only causes death many years later. Most individuals experience a primary infection resulting in a febrile illness about 2-4 weeks after exposure. This illness coincides with sero-conversion. The symptoms are similar to those of glandular fever, namely fever, sore throat, night sweats, lymphadenopathy, diarrhea. Following the primary infection, the patient enters a stage of clinical latency. During this time the patient feels fine, but they are infectious as they have on-going viral replication and have HIV antibodies in their blood. As the CD4 counts drop, there is a gradual onset of a variety of prodromal disorders, such as weight loss, fever, persistent lymphadenopathy, oral candidiasis and diarrhea. These symptoms precede the progression to AIDS.

Anti-retroviral therapeutics consist of several class of drugs which may be used in combination with each other and with galectin-9 as described herein. Use of these drugs in combination can be termed anti-retroviral therapy (ART), combination anti-retroviral therapy (cART) or highly active anti-retroviral therapy (HAART). Anti-retroviral therapeutics are broadly classified by the phase of the retrovirus life-cycle that the drug inhibits.

Entry inhibitors (or fusion inhibitors) interfere with binding, fusion and entry of HIV-1 to the host cell by blocking one of several targets. Maraviroc and enfuvirtide are the two currently available agents in this class. Maraviroc works by targeting CCR5, a co-receptor located on human helper T-cells. Caution should be used when administering this drug however due to a possible shift in tropism which allows HIV to target an alternative co-receptor such as CXCR4. In rare cases, individuals may have a mutation in the CCR5 delta gene which results in a nonfunctional CCR5 co-receptor and in turn, a means of resistance or slow progression of the disease. However as mentioned previously, this can be overcome if an HIV variant that targets CXCR4 becomes dominant. To prevent fusion of the virus with the host membrane, enfuvirtide can be used. Enfuvirtide is a peptide drug that must be injected and acts by interacting with the N-terminal heptad repeat of gp41 of HIV to form an inactive hetero six-helix bundle, therefore preventing infection of host cells.

Nucleoside reverse transcriptase inhibitors (NRTI) and nucleotide reverse transcriptase inhibitors (NtRTI) are nucleoside and nucleotide analogues which inhibit reverse transcription. HIV is an RNA virus and hence unable to become integrated into the DNA in the nucleus of the human cell; it must be "reverse" transcribed into DNA. Since the conversion of RNA to DNA is not done in the mammalian cell it is performed by a viral protein which makes it a selective target for inhibition. NRTIs are chain terminators such that once incorporated, work by preventing other nucleosides from also being incorporated into the DNA chain because of the absence of a 3' OH group. Both act as competitive substrate inhibitors. Examples of currently used NRTIs include zidovudine, abacavir, lamivudine, emtricitabine, and tenofovir.

Non-Nucleoside reverse transcriptase inhibitors (NNRTI) inhibit reverse transcriptase by binding to an allosteric site of the enzyme; NNRTIs act as non-competitive inhibitors of reverse transcriptase. NNRTIs affect the handling of substrate (nucleotides) by reverse transcriptase by binding near the active site. NNRTIs can be further classified into 1st generation and second generation NNRTIs. 1st generation NNRTIs include nevirapine and efavirenz. Second generation NNRTIs are etravirine and rilpivirine. HIV-2 is naturally resistant to NNRTIs.

Integrase inhibitors (also known as integrase nuclear strand transfer inhibitors or INSTIs) inhibit the viral enzyme integrase, which is responsible for integration of viral DNA into the DNA of the infected cell. There are several integrase inhibitors currently under clinical trial, and raltegravir became the first to receive FDA approval in October 2007. Raltegravir has two metal binding groups that compete for substrate with two Mg2+ ions at the metal binding site of integrase. Recently, two other clinically approved integrase inhibitors are elvitegravir and dolutegravir.

Protease inhibitors block the viral protease enzyme necessary to produce mature virions upon budding from the host membrane. Particularly, these drugs prevent the cleavage of gag and gag/pol precursor proteins. Virus particles produced in the presence of protease inhibitors are defective and mostly non-infectious. Examples of HIV protease inhibitors are Lopinavir, Indinavir, Nelfinavir, Amprenavir and Ritonavir. Darunavir and atazanavir are currently recommended as first line therapy choices. Maturation inhibitors have a similar effect by binding to gag, but development of two experimental drugs in this class, Bevirimat and Vivecon, was halted in 2010. Resistance to some protease inhibitors is high. Second generation drugs have been developed that are effective against otherwise resistant HIV variants

Combinations of antiretrovirals create multiple obstacles to HIV replication to keep the number of offspring low and reduce the possibility of a superior mutation. If a mutation that conveys resistance to one of the drugs being taken arises, the other drugs continue to suppress reproduction of that mutation. With rare exceptions, no individual antiretroviral drug has been demonstrated to suppress an HIV infection for long; these agents must be taken in combinations in order to have a lasting effect. As a result, the standard of care is to use combinations of antiretroviral drugs. Combinations usually comprise of three drugs from at least two different classes. This three drug combination is commonly known as a triple cocktail. Combinations of antiretrovirals are subject to positive and negative synergies, which limits the number of useful combinations. Additionally, there are now several options that combine three drugs into one pill taken once daily.

| **Brand Name** | **Drug Combination** |
|---|---|
| Combivir | zidovudine + lamivudine |
| Kaletra | lopinavir + ritonavir |
| Trizivir | abacavir + zidovudine + lamivudine |
| Epzicom /Kivexa | abacavir/lamivudine |
| Truvada | tenofovir/emtricitabine |
| Atripla | efavirenz + tenofovir/emtricitabine |
| Complera | rilpivirine + tenofovir/emtricitabine |
| Stribild | elvitegravir + cobicistat + tenofovir/emtricitabine |
| Triumeq | dolutegravir + abacavir/lamivudine |

As described in Example 8 and Figure 11 herein, other therapeutic agents may be useful in combination with galectin-9 of the invention, such as a thienotriazolodiazepine (e.g., JQ1), which is a potent inhibitor of the BET family of bromodomain proteins which include BRD2, BRD3, BRD4, for example.

Galectin-9 is member of the galectin family and host determinant of HIV pathogenesis. The galectin family consists of a group of glycan-binding animal lectins characterized by conserved carbohydrate recognition domains (CRDs), defined by shared consensus amino acid sequences which confer specific binding to 13- galactoside-containing glycoconjugate proteins. Galectins are ubiquitously expressed throughout the animal kingdom, from lower organisms, such as nematodes and sponges, to higher mammalian species, such as humans. The presence of galectins across many species coupled with the high conservation of amino acid residues essential for ligand recognition in the CRD suggests that galectins are involved in critical, conserved biological processes. Fifteen members of the mammalian galectin family have been identified to date. Proto-type galectins (galectins-1, 2, 5, 7, 10, 11, 13, 14, and 15) contain a single CRD with a short N-terminal sequence, while tandem-repeat-type galectins (galectins-4, 6, 8, 9, and 12) include two non-identical CRDs joined by a short linker peptide sequence. The single chimera-type galectin (galectin-3) has one CRD with an extended N terminus containing several repeats of a proline-tyrosine-glycine-rich motif. Galectin-9 (Gal-9), a tandem-repeat-type galectin, was originally isolated from mouse embryonic kidney cells and was later found to be widely distributed throughout rat and mouse tissues. In contrast, expression of human Gal-9 is restricted to peripheral blood leukocytes and lymphatic tissues. Gal-9 is a member of the 13-galactoside-binding animal lectin family that has been recognized to play an important role in HIV pathogenesis through regulation of both adaptive and innate defense mechanisms. Gal-9 acts as a cytokine and can modulate the activity and function of several immune cell types. Galectins that are present in the extracellular environment can modulate signaling pathways by regulating receptor lattice formation on the cell membrane. Multiple reports suggest that Gal-9 modulates the expression of p21, and either induces or suppresses p21 expression in a context-dependent manner. Recombinant stable Gal-9 (up to 100pg daily dosage) has been used successfully and safely as therapy in a number of mouse disease models including graft versus host disease, rheumatoid arthritis, and asthma. Gal-9 delays the progression of leukemia and colon cancer in mice by inducing apoptosis, and by blocking metastasis, respectively. Manipulating Gal-9 levels directly through administration of recombinant Gal-9, or indirectly by interfering with secretory pathways may represent novel therapeutic approaches to enhance reactivation of HIV latently infected cells.

Several cell-intrinsic immune factors have been discovered that restrict HIV replication in the absence of antiretroviral drugs. These factors include BST-2/tetherin, p21, schlafen 11, SAMHDI, the PAF1 complex, and members of the TRIM and APOBEC3 cytidine deaminase families. The relevance of restriction factors to the control of HIV in chronically-infected individuals undergoing interferon-a/ribavirin therapy, and in HIV elite controllers, individuals who maintain undetectable viremia in the absence of ART has been demonstrated. Recent reports have demonstrated associations between the expression levels of cell-intrinsic antiviral factors and HIV reservoir size and transcriptional activity in elite controllers.

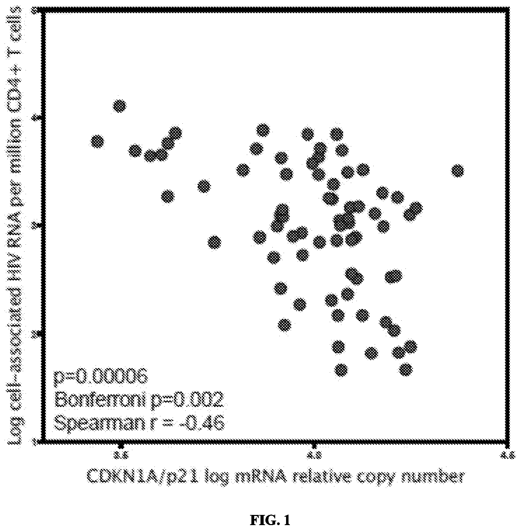

It was recently reported that expression of the p21 (CDKN1A/CIP1/WAF1) host restriction factor exhibits a highly significant negative correlation with HIV latent reservoir size in individuals on suppressive ART (as measured by CD4+ T cell-associated HIV RNA). This suggests that p21 may contribute to the control of viral expression and ongoing replication during ART (Figure 1). The p21 cyclin-dependent kinase (Cdk) inhibitor has been associated with the control or repression of HIV *in vitro,* and with the natural control of HIV *in vivo.* p21 inhibits trans-activator protein (Tat)-mediated HIV transcription by suppressing the activity of the positive transcription elongation factor (P-TEFb) in the host cell. Therefore, in the setting of complete ART-mediated virologic suppression, the inverse relationship between p21 expression and CD4+ T cell-associated HIV RNA that was observed likely reflects p21-enforced repression of HIV proviral transcription. This seminal observation by our group led us to the hypothesis that inhibition of p21 expression promotes reversal of HIV latency *in vivo,* and p21 may therefore represent a promising pharmacological target for HIV eradication efforts. A survey of the p21 literature revealed that the human protein "galectin-9" (Gal-9) is known to modulate the expression of the p21 protein. Based on extensive *in vivo* gene expression profiling experiments and multiple published reports describing the regulation of p21 by Gal-9, it was sought to determine if Gal-9 treatment of HIV latently-infected cells would reverse HIV latency and induce viral expression.

Therefore, in one embodiment, the present invention provides a method of reversing human immunodeficiency virus (HIV) latency in a cell comprising administering a galectin protein to a subject in need thereof, thereby reversing the HIV latency. In one aspect of the disclosure, the galectin protein is galectin-1, galectin-2, galectin-3, galectin-4, galectin-5, galectin-6, galectin-7, galectin-8, galectin-10, galectin-11, galectin-12, galectin-13, galectin-14 or galectin-15. In a specific aspect of the invention, the galectin protein is galectin-9, for example recombinant galectin-9 (rGal-9). In another aspect, after administration of the galectin protein the HIV is hypermutated. In a further aspect, the hypermutated HIV is replication incompetent. In an additional aspect, expression of at least one APOBEC3 cytidine deaminase and at least one NFκB pathway component is induced. In one aspect, the induction of expression of the at least one APOBEC3 cytidine deaminase and the at least one NFκB pathway component is at least two fold above normal physiologic levels. In specific aspects, the at least one APOBEC3 cytidine deaminase is APOBEC3B, APOBEC3F, APOBEC3G, APOBEC3H or a combination thereof and the at least one NFκB pathway component is NFκB1, NFκB2, NFκBIA, NFκBID, NFκBIE, NFκB1, NFκBIL1, NFκBIZ, NFRκB, REL, RELA, RELB or a combination thereof. In another aspect, the expression of at least one histone deacetylase (HDAC) protein is decreased compared to normal physiologic levels. In specific aspects, the at least one HDAC protein is HDAC1, HDAC2, HDAC3 or a combination thereof.

In a further embodiment, the present disclosure provides a method of treating a subject with a retrovirus infection comprising administering recombinant galectin-9 (rGal-9) to the subject, thereby treating the retroviral infection. In one aspect, the retrovirus is HIV-1, HIV-2, HTLV-1, HTLV-2, HTLV-3, HTLV-4 or a combination thereof. In a specific aspect, the retrovirus is HIV-1 or HIV-2. In another aspect, after administration of rGal-9 the HIV is hypermutated. In a further aspect, the hypermutated HIV is replication incompetent. In an additional aspect, expression of at least one APOBEC3 cytidine deaminase and at least one NFκB pathway component is induced. In one aspect, the induction of expression of the at least one APOBEC3 cytidine deaminase and the at least one NFκB pathway component is at least two fold above normal physiologic levels. In specific aspects, the at least one APOBEC3 cytidine deaminase is APOBEC3B, APOBEC3F, APOBEC3G, APOBEC3H or a combination thereof and the at least one NFκB pathway component is NFκB1, NFκB2, NFκBIA, NFκBID, NFκBIE, NFκB1, NFκBIL1, NFκBIZ, NFRκB, REL, RELA, RELB or a combination thereof. In another aspect, the expression of at least one histone deacetylase (HDAC) protein is decreased compared to normal physiologic levels. In specific aspects, the at least one HDAC protein is HDAC1, HDAC2, HDAC3 or a combination thereof. In a further aspect, an anti-retroviral therapeutic is administered. In one aspect, the anti-retroviral therapeutic is enfuvirtide, zidovudine, abacavir, lamivudine, emtricitabine, tenfovir, nevirpine, efavirenz, etravirine, rilpivirine, raltegravir, elvitegravir, dolutegravir, lopinavir, indinavir, nelfinavir, amprenavir, ritonavir, darunavir, atazanavir, bevirimat, vivecon, stavudine, didanosine, delavirdine, nevirapine, fosamprenavir, saquinavir, tipranavir, maraviroc or a combination thereof. In another aspect, the anti-retroviral therapeutic is a nucleoside/nucleotide reverse transcriptase inhibitor (NRTI), non-nucleoside reverse transcriptase inhibitor (NNRTI), protease inhibitor, fusion or entry inhibitor, integrase inhibitor or a combination thereof.

In an additional embodiment, the present invention provides pharmaceutical compositions comprising a recombinant galectin protein and a pharmaceutically acceptable carrier. In one aspect of the disclosure, the recombinant galectin protein is galectin-1, galectin-2, galectin-3, galectin-4, galectin-5, galectin-6, galectin-7, galectin-8, galectin-10, galectin-11, galectin-12, galectin-13, galectin-14 or galectin-15. In a specific aspect of the invention, the recombinant galectin protein is galectin-9 (rGal-9).

Pharmaceutical compositions comprising the recombinant proteins described herein are provided. Such compositions can comprise a therapeutically effective amount of a recombinant galectin protein with one or more additional components such as a pharmaceutically acceptable carrier, excipient, or diluent. Such additional components can include buffers, carbohydrates, polyols, amino acids, chelating agents, stabilizers, and/or preservatives, among many possibilities.

The pharmaceutical compositions of the present invention may be administered by a parenteral route, for example by injection. Subcutaneous, intramuscular, intravenous, intraarterial, intralesional, and peritoneal bolus injections are possible routes of administration. The pharmaceutical compositions can also be administered via infusion, for example intravenous or subcutaneous infusion. Topical administration is also possible, especially for diseases involving the skin. Alternatively, the pharmaceutical compositions can be administered through contact with a mucus membrane, for example by intra-nasal, sublingual, vaginal, or rectal administration or administration as an inhalant. Alternatively, certain appropriate pharmaceutical compositions can be administered orally.

As shown in the Examples, treatment with recombinant, stable form Gal-9 protein (rGal-9), potently reverses HIV latency, to a degree that is substantially higher than any existing established latency reversal agents that have been evaluated in clinical trials. Moreover, it is shown that rGal-9 treatment strongly induces the expression of APOBEC3 host restriction factors which severely mutagenize, or "hypermutate" HIV. This induction will ensure that any reactivated viruses are genetically eroded to the point that they will be non-infectious, minimizing the possibility that therapeutic latency reversal will reseed the latent pool. The data presented here demonstrate that treatment with recombinant, stable form Gal-9 protein (rGal-9), potently reverses HIV latency and strongly induces the expression of the APOBEC3 host restriction factors which severely hypermutate HIV. Therefore galectins or galectin pathway components are likely to prove useful in HIV curative strategies. Moreover, it is anticipated that rGal-9 will exhibit similar capacities to reactivate and hypermutate all retroviruses, including other viruses that are pathogenic in humans (e.g. HTLV-I).

The invention in all its aspects is illustrated further in the following Examples. The Examples do not, however, limit the scope of the invention, which is defined by the appended claims.

### EXAMPLES

### EXAMPLE 1

### Potent Reversal of HIV-1 Latency using Galactin-9

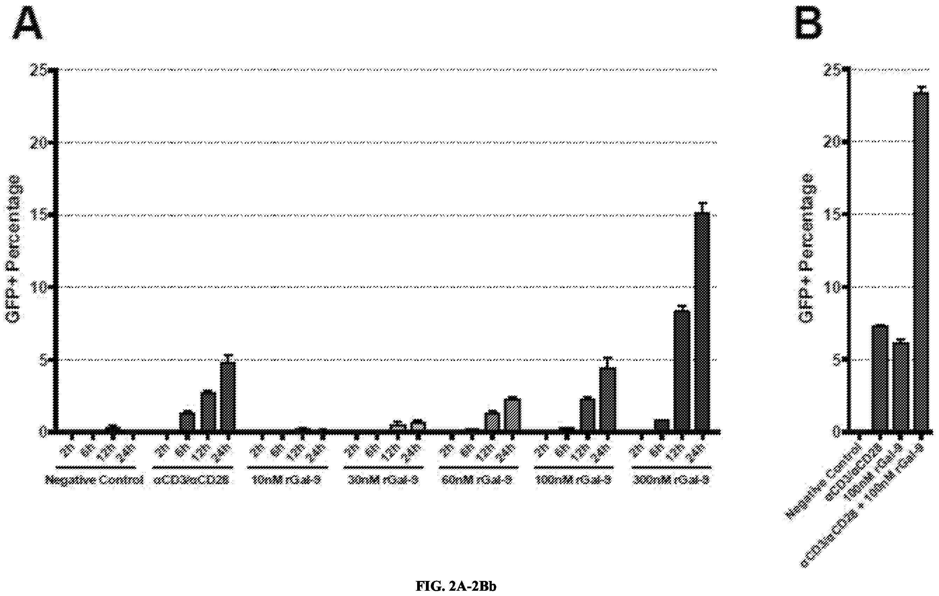

It was hypothesized that rGal-9 would have a significant effect on reactivation of latent HIV. To address this hypothesis, experiments were performed in the established "J-Lat" model of HIV latency. J-Lat cells harbor latent, transcriptionally competent HIV provirus that encodes green fluorescent protein (GFP) as an indicator of reactivation Dose-response experiments were performed by stimulating J-Lat 5A8 cells (a J-Lat variant that is sensitive to anti-CD3/CD28 stimulation) with recombinant Gal-9 for increasing periods of time. The data revealed that rGal-9 reactivated latently infected J-Lat cells in a dose-dependent manner, and with potency equivalent to or greater than that seen with CD3/CD28 stimulation alone (Figure 2A). Simultaneous rGal-9 and CD3/CD28 stimulation were synergistic, resulting in a nearly three-fold increase in the percentage of J-Lat cells expressing HIV RNA (represented by GFP expression) compared to CD3/CD28 stimulation alone. (Figure 2B). *In vitro* reactivation of HIV expression by recombinant galectin-9 in the J-Lat latency model. J-Lat cells were analyzed by flow cytometry to assess mean fluorescence intensity of HIV-encoded GFP expression after reactivation with recombinant Galactin-9 (rGal-9). (A) Dose response of HIV reactivation by rGal-9 after 2, 6, 12, and 24 hours of stimulation. Anti-CD3/CD28 antibodies conjugated to beads were used as a positive control. (B) Effects of combining 100nM of rGal-9 and aCD3/aCD28 stimulation after 24 hours of stimulation. Results are mean ± SD from a representative experiment performed in triplicate.

### EXAMPLE 2

### Effects of rGal-9 treatment on the host transcriptome

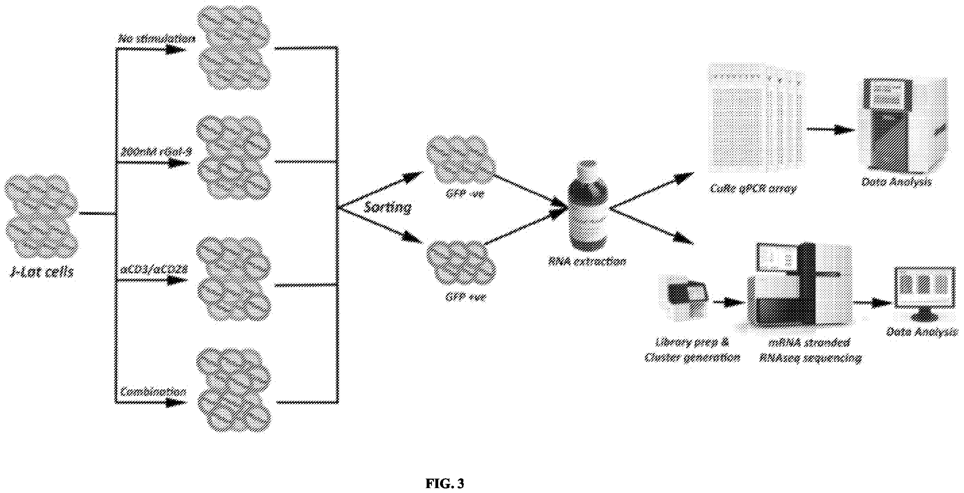

In addition to evaluating the effects of rGal-9 on reactivation of latent HIV, it was sought to determine the effects of rGal-9 on the human transcriptome, with a particular focus on (1) expression of genes involved in HIV transcription and latency, and (2) immune genes of relevance to control of HIV and clearance of HIV-infected cells. A two-pronged approach was used; "CuRe" (Cumulative Restriction) quantitative PCR array was used to specifically measure intrinisic immune gene expression, and RNA-sequencing was used to comprehensively assess global effects on gene expression (Figure 3). As part of workflow, GFP-positive and GFP-negative cells contain reactivated (transcriptionally active) HIV proviruses and latent (transcriptionally inactive) proviruses, respectively.

### EXAMPLE 3

### rGal-9 treatment induces anti-HIV cell-intrinsic immunity, including the APOBEC3 cytidine deaminases

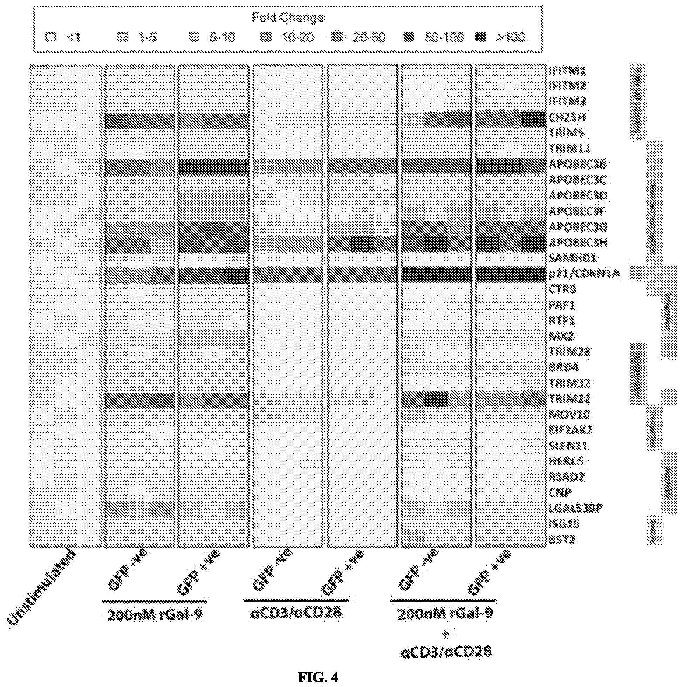

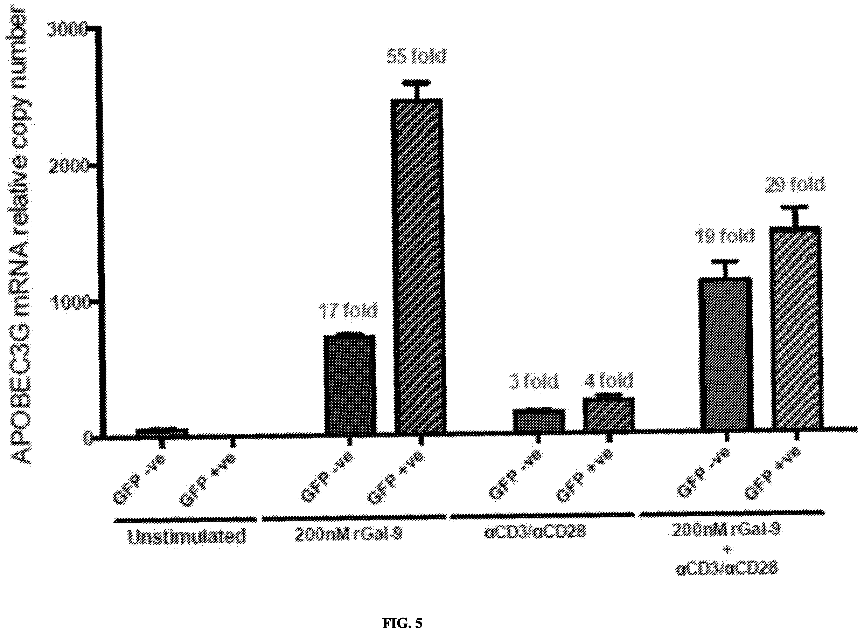

The gene expression data was examined to determine how rGal-9 modulates anti-HIV cell-intrinsic immune defenses (Figure 4). Effects on 42 established anti-HIV restriction factors were examined, as described in multiple papers (Abdel-Mohsen et al. (2014) J Virol 88:763 and Abdel-Mohsen (2013) Retrovirology 10:106). A number of factors that inhibit HIV prior to integration in the host genome were significantly induced, while factors that suppress HIV post-integration were not induced. This is an ideal scenario for an agent to be used within the shock-and-kill HIV eradication framework. Of particular relevance, it was determined that the APOBEC3 cytidine deaminases, including APOBEC3B, APOBEC3F, APOBEC3G, and APOBEC3H, were potently induced by rGal-9 in CD4+ T cells. Based on the known mechanism of action associated with these APOBEC3 factors, this suggests that any virus that is reactivated by rGal-9 will be severely hypermutated upon infection of a new cell, rendering all produced virus replication incompetent. This is highly relevant to the shock-and-kill HIV cure framework, as it is becoming increasingly clear that ART does not completely block viral replication, especially in tissues with suboptimal drug penetration. The human cytidine deaminases APOBEC3G and APOBEC3F serve as innate antiviral defense mechanisms by introducing C-to-U changes in the minus strand DNA of retroviruses and hepadnaviruses during replication (resulting in G-to-A mutations in the genomic sense strand sequence). The HIV-1 genome, however, encodes the 23 kilodalton protein Vif (virion infectivity factor) which specifically counteracts this defense by promoting the proteolytic degradation of APOBEC3 in the host cell. In the absence of Vif expression, APOBEC3 is incorporated into virions and the viral genome undergoes extensive G-to-A mutation in the coding strand, or "hypermutation", typically rendering it non-viable within a single replicative cycle. Given the levels of APOBEC3G induction (up to 55-fold) that were observed in response to rGal-9 (Figure 4, Figure 5), it was expected that the antagonistic effects of the viral Vif protein will be neutralized, reactivated virus will package high concentrations of APOBEC3G, and all progeny virions will be rendered replication incompetent. This scenario is similar to published observations by our group and others suggesting that induction of APOBEC3G to supraphysiologic levels (even just at the two- to three-fold level) potently neutralize HIV-1 and other retroviruses *in vivo.* Figure 4 shows a heat map describing effects of rGal-9 on anti-HIV cell-intrinsic immunity. The data represented were collected using a custom quantitative PCR array. Observations were mirrored in our RNA-sequencing data, validating the patterns observed. Fold-change with respect to baseline (pre-treatment) conditions are represented. Figure 5 shows the induction of the APOBEC3G host cytidine deaminase in HIV latently-infected cells by rGal-9. GFP-positive and GFP-negative cells contain reactivated (transcriptionally active) HIV proviruses and latent (transcriptionally inactive) proviruses are shown. Mean ± SEM are represented.

### EXAMPLE 4

### rGal-9 treatment induces NFkB pathway components

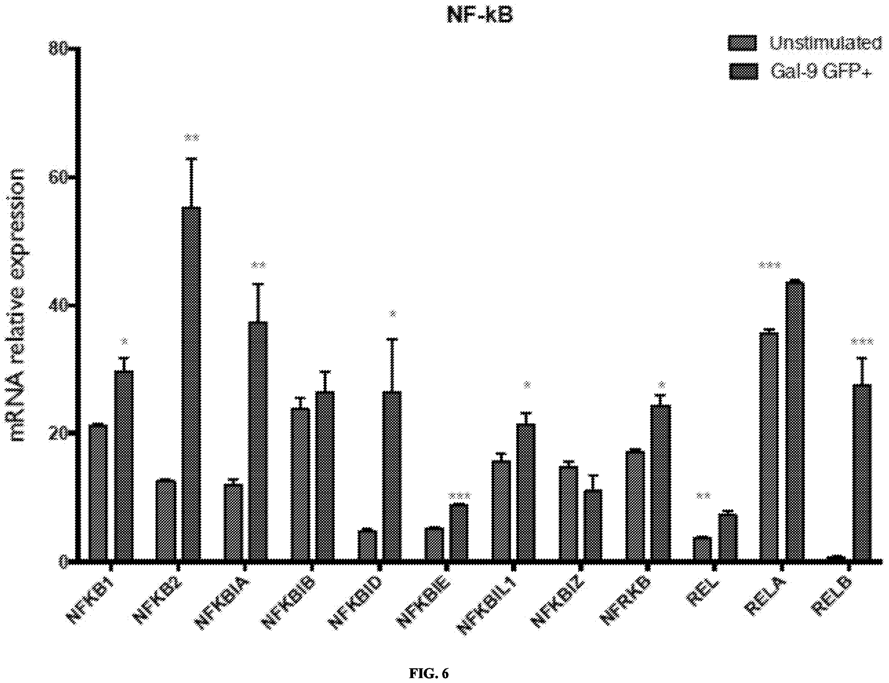

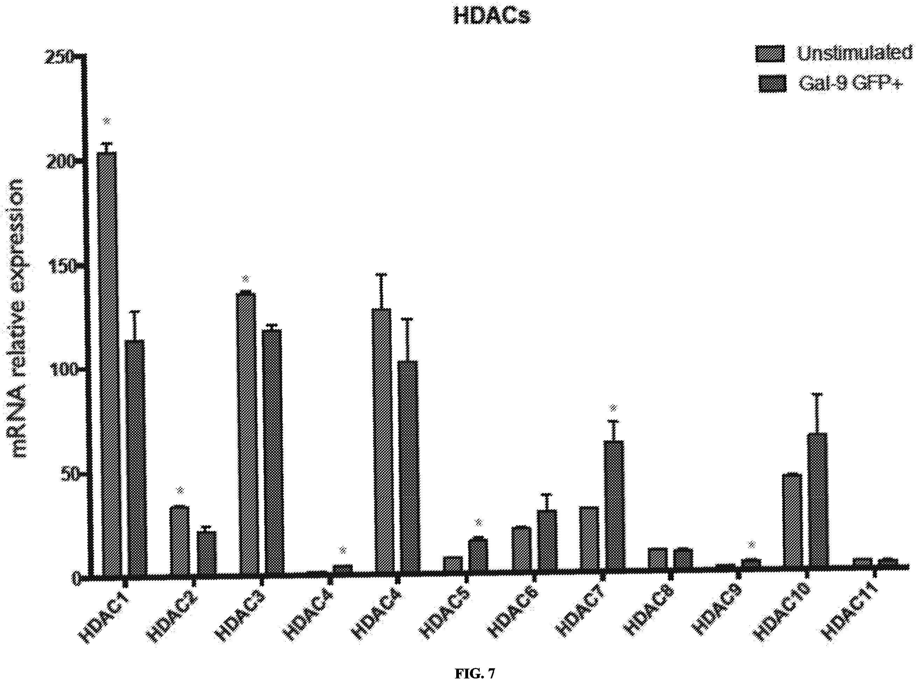

The nuclear factor kappa-light-chain-enhancer of activated B cells (NFkB) pathway is known to be critical to HIV-1 transcription. The NFkB pathway is central to the signal-dependent activation of HIV transcription initiation, and enables recruitment of histone acetyltransferases (HATs) to the viral long terminal repeat (LTR) promoter. We therefore hypothesized that real-9 treatment would induce multiple components of the NFkB pathway, contributing to its potent enhancing effects on HIV-1 transcription. As expected, analyses of our RNA-seq data unequivocally demonstrate that several NFkB pathway genes are induced by NFkB treatment (Figure 6). The data were generated using RNA-seq. Mean ± SD represented in bar graph. Asterisks indicate statistically significant differences based on a t-test, correcting for false discovery rate: * = FDR<0.05, ** = FDR<0.01,***= FDR<0.001. As shown in Figure 7, rGal-9 selectively inhibits histone deacetylases (HDACs). The data were generated using RNA-seq. Mean ± SD represented in bar graph. Asterisks indicate statistically significant differences based on a t-test, correcting for false discovery rate: * = FDR<0.05.

### EXAMPLE 5

### rGal-9 has minimal effects on viability of primary, freshly-collected cells

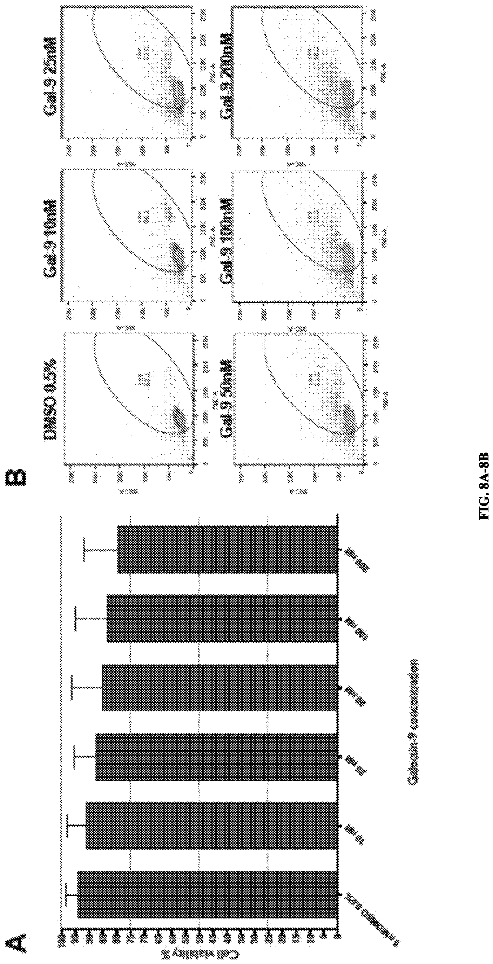

In addition to evaluating the effects of rGal-9 on reactivation of latent HIV, it was sought to evaluate the toxicity of real-9 and determine the effects of rGal-9 on the viability of primary cells. Despite the ability of rGal-9 to potently reactivate latent HIV, its potential application in vivo would be undermined by significant, indiscriminate impact on cellular viability. 100 cc of fresh blood were obtained from multiple healthy controls, collected from HIV-uninfected donors through the healthy donor program at Blood Systems Research Institute. The experiment clearly demonstrates that the effects of rGal-9 on the viability of primary peripheral blood mononuclear cells (PBMC) and purified CD4+ T cells is minimal, further reinforcing the potential human application of rGal-9 (Figure 8) Escherichia coli (Epi300, Epicentre) were grown on Luria broth or agar supplemented with chloramphenicol (20 mg L-1) or kanamycin (50 mg L-1) or ampicillin (50 mg L-1) or tetracycline (10 mg L-1) or gentamicin (20 mg L-1) or combinations of these as needed. As shown in Figure 8, freshly isolated primary CD4+ T cells tolerate rGAL-9 treatment at least up to a concentration of 200nM. Figure 8A shows the percentage of live CD4+ T cells from three healthy donors treated with either 0.5% DMSO or escalating concentrations of galectin-9 for 24 hours. Figure 8B shows a representative set of the flow cytometry data. The dot plots are FSC-SSC plots. FSC and SSC indicate forward scatter and side scatter, respectively

### EXAMPLE 6

### Potent ex vivo reactivation of latent HIV by rGal-9

CD4+ T cells were isolated from three ART-suppressed HIV-infected individuals. Cells were either untreated or treated with DMSO 0.5% (negative control), aCD3/aCD28 beads (1:1 cells to beads ratio), PMA (5nM) / ionomycin (1pM), SAHA(1pM), or different concentrations of rGal-9 (200nM, 500nM, 1pM, and 1.65pM) for 24 hours. Cells were also pulse-treated with romidepsin (40nM) for 4 hours, washed twice and cultured for 5 days (per the Gilead recommended protocol). Cell-associated total RNA was extracted using Qiagen Allprep RNA/DNA/protein kit with on-column DNase treatment. HIV cell-associated RNA levels were quantified using quantitative PCR (qPCR) targeting the highly conserved LTR region, run in triplicate. Fold increase in cell-associated HIV RNA was determined relative to the corresponding DMSO treated control for each individual time point. The fold change for each donor and condition is based on mean number of HIV copies from two independent measurements. Boxes represent range and lines represent median values. CD4+ T cells were isolated from three ART-suppressed HIV-infected individuals. Cell-associated total RNA was extracted using Qiagen Allprep RNA/DNA/protein kit with on-column DNase treatment. HIV cell-associated RNA levels were quantified using quantitative PCR (qPCR) targeting the highly conserved LTR region, run in triplicate. Fold increase in cell-associated HIV RNA was determined relative to the corresponding DMSO treated control for each individual time point. The fold change for each donor and condition is based on mean number of HIV copies from two independent measurements. Boxes represent range and lines represent median values.

### EXAMPLE 7

### The manipulation of cell-surface glycans to reverse HIV latency

Experiments have been performed to identify the mechanism of action associated with the reversal of HIV latency mediated by recombinant galectin-9 (rGal-9). By selectively depleting glycans from the surface of infected cells, we have determined that the presence of particular cell surface glycans (rather than previously recognized protein ligands of galectin-9) is essential to the ability of rGal-9 to reverse HIV latency (see Figure 10). The results show that glycans play a critical role in rGal-9 signaling. Figures 10A-D show graphs and a flow cytometry analysis to show rGal-9 induces HIV transcription and reactivation in a glycan-dependent manner. Figure 10A- Effects of anti-Tim-3 antibody, anti-CD44 antibody, or anti-PDI antibody administration on rGal-9-mediated reactivation of HIV in J-Lat 5A8 cells. Antibodies were added 30 minutes prior to administration of 200nM rGal-9. α-lactose was used as a positive control. Figures 10B, C- Treatment of J-Lat 5A8 cells with either 1 µg/ml tunicamycin, or with an enzymatic deglycosylation mix for 24 h prior to rGal-9 stimulation. J-Lat cells were analyzed by flow cytometry to assess HIV-encoded GFP expression. Statistical comparisons were performed using two-tailed Mann-Whitney tests. Figure 10D- Effects of deglycosylation enzyme combinations on rGal-9-mediated HIV latency reversal in J-Lat 5A8 cells. N = PNGase F (Elizabethkingia miricola); O = O-Glycosidase (recombinant from Streptococcus pneumonia; S = α-(2→3,6,8,9)-Neuraminidase (recombinant from Arthrobacter ureafaciens); B = β(1→4)-Galactosidase (recombinant from Streptococcus pneumonia) + β-N-Acetylglucosaminidase (recombinant from Streptococcus pneumonia). Mean ± SEM is displayed.

By selectively depleting glycans from the surface of infected cells, we have determined that the presence of particular cell surface glycans is important to the ability of rGal-9 to reverse HIV latency (see Figure 10). Glycans therefore play a role in rGal-9 signaling. Thus, by modulating host cellular glycosylation one can achieve HIV latency reversal. Glycosylation modulating agents may be administered prior to, simultaneously with or following galectin-9 treatment.

### EXAMPLE 8

### Utilization of galectins in synergistic combinations with other latency reversal agents, including bromodomain inhibitors

This Example shows the use of rGal-9 in synergistic combinations with other latency reversal agents to achieve a treatment or cure for HIV infection. This Example provides data supporting this concept. For example, rGal-9 co-administration with the latency reversal agent "JQ1", a bromodomain inhibitor, exhibits synergistic activity (see Figure 11). These data that illustrate that rGal-9 will likely exhibit synergistic activity with other classes of HIV latency reversal agents, and will be well-suited to incorporation into multi-drug cocktails to cure HIV infection.

Figures 11A and 11B provide data showing that Galectin-9 synergizes with JQ1 in reactivating latent HIV. Figure 11A- CD4+ T cells from HIV-infected ART-suppressed individuals were treated with 500nM of rGal-9, 1 µM vorinostat, 10nM bryostatin, 300nM prostratin, 1 µM JQ1, or 30nM panobinostat alone or in combination with 500nM of rGal-9 for 24 hours, and fold induction of cell-associated HIV RNA was determined using quantitative real-time PCR. * = p<0.05 compared with Gal-9 500nM treatment alone. Figure 11 B- The Bliss independence model was utilized for calculation of synergy for drug combinations. Δfaxy = 0 signifies pure additive effect. Δfaxy>0 signifies Synergy, while Δfaxy<0 signifies antagonism. Statistical significance was calculated using a one tailed paired t-test comparing predicted and observed drug combination effects. * = p < 0.05.

## Claims

1. A galectin-9 protein for use in a method of treating HIV infection by reversing human immunodeficiency virus (HIV) latency in a cell, the method comprising administering the galectin protein to a subject.

2. The galectin-9 protein for use according to claim 1, wherein the galectin-9 protein is a recombinant galectin-9 (rGal-9).

3. The galectin-9 protein for use according to claim 1, wherein after administration of the galectin-9 protein the HIV is hypermutated, optionally wherein the hypermutated HIV is replication incompetent.

4. The galectin-9 protein for use according to claim 1, wherein expression of at least one APOBEC3 cytidine deaminase and at least one NFκB pathway component is induced, optionally wherein the induction of expression of the at least one APOBEC3 cytidine deaminase and the at least one NFκB pathway component is at least twofold above normal physiologic levels.

5. The galectin-9 protein for use according to claim 4, wherein the at least one APOBEC3 cytidine deaminase is selected from the group consisting of APOBEC3B, APOBEC3F, APOBEC3G, APOBEC3H, or any combination thereof, and/or wherein the at least one NFκB pathway component is selected from the group consisting of NFκB1, NFκB2, NFκBIA, NFκBID, NFκBIE, NFκB1, NFκBIL1, NFκBIZ, NFRκB, REL, RELA, RELB, or any combination thereof.

6. The galectin-9 protein for use according to claim 1, wherein expression of at least one histone deacetylase (HDAC) protein is decreased compared to normal physiologic levels, optionally wherein the at least one HDAC protein is selected from the group consisting of HDAC1, HDAC2, HDAC3, or any combination thereof.

7. The galectin-9 protein for use according to claim 1, wherein the HIV is selected from the group consisting of HIV-1 and HIV-2 or a combination thereof.

8. The galectin-9 protein for use according to claim 1, wherein the method further comprises administration of an anti-retroviral or additional therapeutic agent, optionally wherein the anti-retroviral therapeutic is selected from the group consisting of enfuvirtide, zidovudine, abacavir, lamivudine, emtricitabine, tenfovir, nevirpine, efavirenz, etravirine, rilpivirine, raltegravir, elvitegravir, dolutegravir, lopinavir, indinavir, nelfinavir, amprenavir, ritonavir, darunavir, atazanavir, bevirimat, vivecon, stavudine, didanosine, delavirdine, nevirapine, fosamprenavir, saquinavir, tipranavir, maraviroc, a nucleoside/nucleotide reverse transcriptase inhibitor (NRTI), non-nucleoside reverse transcriptase inhibitor (NNRTI), protease inhibitor, fusion or entry inhibitor, integrase inhibitor, or any combination thereof.

9. A pharmaceutical composition comprising a galectin-9 protein and a pharmaceutically acceptable carrier for use in a method of treating HIV infection by reversing human immunodeficiency virus (HIV) latency in a cell.

10. The pharmaceutical composition for use of claim 9, wherein the pharmaceutical composition further comprises at least one inhibitor of a BET family bromodomain protein.

11. The pharmaceutical composition for use of claim 10, wherein the at least one inhibitor of a BET family bromodomain protein is JQ1.

12. The galectin-9 protein for use according to claim 1, wherein the method further comprises administering to an HIV infected cell an agent for modulating the infected cell's glycosylation.

## Patentansprüche

1. Galectin-9-Protein zur Verwendung bei einem Verfahren zur Behandlung einer HIV-Infektion durch das Umkehren der Latenz eines humanen Immundefizienz-Virus (HIV) in einer Zelle, wobei das Verfahren das Verabreichen des Galectin-Proteins an ein Individuum umfasst.

2. Galectin-9-Protein zur Verwendung nach Anspruch 1, wobei das Galectin-9-Protein ein rekombinantes Galectin-9 (rGal-9) ist.

3. Galectin-9-Protein zur Verwendung nach Anspruch 1, wobei das HIV nach der Verabreichung des Galectin-9-Proteins hypermutiert ist, wobei das hypermutierte HIV gegebenenfalls replikationsinkompetent ist.

4. Galectin-9-Protein zur Verwendung nach Anspruch 1, wobei die Expression wenigstens einer APOBEC3-Cytidin-Deaminase und wenigstens einer Komponente des NFκB-Pfades induziert wird, wobei die Induktion der Expression der wenigstens einen APOBEC3-Cytidin-Deaminase und der wenigstens einen Komponente des NFκB-Pfades gegebenenfalls wenigstens um das Zweifache über normalen physiologischen Spiegeln liegt.

5. Galectin-9-Protein zur Verwendung nach Anspruch 4, wobei die wenigstens eine APOBEC3-Cytidin-Deaminase ausgewählt ist aus der Gruppe bestehend aus APOBEC3B, APOBEC3F, APOBEC3G, APOBEC3H oder jeder beliebigen Kombination davon und/oder wobei die wenigstens eine Komponente des NFκB-Pfades ausgewählt ist aus der Gruppe bestehend aus NFκB1, NFκB2, NFκBIA, NFκBID, NFKBIE, NFκB1, NFκBIL1, NFKBIZ, NFKKB, REL, RELA, RELB oder jeder beliebigen Kombination davon.

6. Galectin-9-Protein zur Verwendung nach Anspruch 1, wobei die Expression wenigstens eines Histon-Deacetylase- (HDAC-)Proteins im Vergleich zu normalen physiologischen Spiegeln verringert ist, wobei das wenigstens eine HDAC-Protein gegebenenfalls ausgewählt ist aus der Gruppe bestehend aus HDAC1, HDAC2, HDAC3 oder jeder beliebigen Kombination davon.

7. Galectin-9-Protein zur Verwendung nach Anspruch 1, wobei das HIV ausgewählt ist aus der Gruppe bestehend aus HIV-1 und HIV-2 oder einer Kombination davon.

8. Galectin-9-Protein zur Verwendung nach Anspruch 1, wobei das Verfahren weiterhin die Verabreichung eines antiretroviralen oder zusätzlichen Therapeutikums umfasst, wobei das antiretrovirale Therapeutikum gegebenenfalls ausgewählt ist aus der Gruppe bestehend aus Enfuvirtid, Zidovudin, Abacavir, Lamivudin, Emtricitabin, Tenfovir, Nevirpin, Efavirenz, Etravirin, Rilpivirin, Raltegravir, Elvitegravir, Dolutegravir, Lopinavir, Indinavir, Nelfinavir, Amprenavir, Ritonavir, Darunavir, Atazanavir, Bevirimat, Vivecon, Stavudin, Didanosin, Delavirdin, Nevirapin, Fosamprenavir, Saquinavir, Tipranavir, Maraviroc, einem Nukleosid/Nukleotid-Reverse-Transkriptase-Inhibitor (NRTI), Nicht-Nukleosid-Reverse-Transkriptase-Inhibitor (NNRTI), Proteaseinhibitor, Fusions- oder Entry-Inhibitor, Integraseinhibitor oder jeder beliebigen Kombination davon.

9. Pharmazeutische Zusammensetzung, die ein Galectin-9-Protein und einen pharmazeutisch annehmbaren Träger umfasst, zur Verwendung bei einem Verfahren zur Behandlung einer HIV-Infektion durch das Umkehren der Latenz eines humanen Immundefizienz-Virus (HIV) in einer Zelle.

10. Pharmazeutische Zusammensetzung zur Verwendung nach Anspruch 9, wobei die pharmazeutische Zusammensetzung weiterhin wenigstens einen Inhibitor eines Bromodomänenproteins der BET-Familie umfasst.

11. Pharmazeutische Zusammensetzung zur Verwendung nach Anspruch 10, wobei der wenigstens eine Inhibitor eines Bromodomänenproteins der BET-Familie JQ1 ist.

12. Galectin-9-Protein zur Verwendung nach Anspruch 1, wobei das Verfahren weiterhin das Verabreichen eines Mittels an eine HIV-infizierte Zelle zur Modulierung der Glycosylierung der infizierten Zelle umfasst.

## Revendications

1. Protéine de galectine 9 pour utilisation dans un procédé de traitement d'une infection par le VIH par réversion de la latence du virus de l'immunodéficience humaine (VIH) dans une cellule, le procédé comprenant l'administration de la protéine de galectine à un sujet.

2. Protéine de galectine 9 pour utilisation selon la revendication 1, où la protéine de galectine 9 est une galectine 9 recombinante (rGal-9).

3. Protéine de galectine 9 pour utilisation selon la revendication 1, où, après administration de la protéine de galectine 9, le VIH est hypermuté, facultativement où le VIH hypermuté est incompétent pour la réplication.

4. Protéine de galectine 9 pour utilisation selon la revendication 1, où l'expression d'au moins une cytidine désaminase APOBEC3 et d'au moins un composant de la voie NFκB est induite, où l'induction d'expression de l'au moins une cytidine désaminase APOBEC3 et de l'au moins un composant de la voie NFκB est facultativement au moins deux fois au-dessus des niveaux physiologiques normaux.

5. Protéine de galectine 9 pour utilisation selon la revendication 4, où l'au moins une cytidine désaminase APOBEC3 est choisie dans le groupe constitué d'APOBEC3B, APOBEC3F, APOBEC3G, APOBEC3H, ou une combinaison quelconque de celles-ci, et/ou l'au moins un composant de la voie NFκB est choisi dans le groupe constitué de NFκB1, NFKB2, NFKBIA, NFKBID, NFKBIE, NFκB1, NFκBIL1, NFκBIZ, NFRκB, REL, RELA, RELB, ou une combinaison quelconque de ceux-ci.

6. Protéine de galectine 9 pour utilisation selon la revendication 1, où l'expression d'au moins une protéine histone désacétylase (HDAC) est diminuée par rapport à des niveaux physiologiques normaux, où l'au moins une protéine HDAC est facultativement choisie dans le groupe constitué de HDAC1, HDAC2, HDAC3, ou une combinaison quelconque de celles-ci.

7. Protéine de galectine 9 pour utilisation selon la revendication 1, où le VIH est choisi dans le groupe constitué de VIH-1 et VIH-2 ou une combinaison de ceux-ci.

8. Protéine de galectine 9 pour utilisation selon la revendication 1, où le procédé comprend en outre l'administration d'un agent thérapeutique antirétroviral ou supplémentaire, l'agent thérapeutique antirétroviral étant facultativement choisi dans le groupe constitué des enfuvirtide, zidovudine, abacavir, lamivudine, emtricitabine, tenfovir, névirpine, éfavirenz, étravirine, rilpivirine, raltégravir, elvitégravir, dolutégravir, lopinavir, indinavir, nelfinavir, amprénavir, ritonavir, darunavir, atazanavir, bévirimat, vivécon, stavudine, didanosine, délavirdine, névirapine, fosamprénavir, saquinavir, tipranavir, maraviroc, un inhibiteur nucléosidique / nucléotidique de la transcriptase inverse (INTI), un inhibiteur non nucléosidique de la transcriptase inverse (INNTI), un inhibiteur de protéase, un inhibiteur de fusion ou d'entrée, un inhibiteur d'intégrase, ou une combinaison quelconque de ceux-ci.

9. Composition pharmaceutique comprenant une protéine de galectine 9 et un véhicule pharmaceutiquement acceptable pour utilisation dans un procédé de traitement d'une infection par le VIH par réversion de la latence du virus de l'immunodéficience humaine (VIH) dans une cellule.

10. Composition pharmaceutique pour utilisation selon la revendication 9, où la composition pharmaceutique comprend en outre au moins un inhibiteur d'une protéine à bromodomaine de la famille BET.

11. Composition pharmaceutique pour utilisation selon la revendication 10, dans laquelle l'au moins un inhibiteur d'une protéine à bromodomaine de la famille BET est JQ1.

12. Protéine de galectine 9 pour utilisation selon la revendication 1, où le procédé comprend en outre l'administration à une cellule infectée par le VIH d'un agent pour moduler la glycosylation de la cellule infectée.
